## Europäisches Patentamt

## European Patent Office

## Office européen des brevets

(19)

(11) Veröffentlichungsnummer: **0 123 035 B1**

(12) **EUROPÄISCHE PATENTSCHRIFT**

(45) Veröffentlichungstag der Patentschrift:
28.01.87

(51) Int. Cl.[4]: **C 07 D 233/88, C 07 C 127/15**

(21) Anmeldenummer: **84101558.9**

(22) Anmeldetag: **15.02.84**

(54) 4-Amino-3-imidazolin-2-on und (2-Methoxy-2-imino-ethyl)-harnstoff, ihre Herstellung und Verwendung.

(30) Priorität: **19.02.83 DE 3305778**

(43) Veröffentlichungstag der Anmeldung:
**31.10.84 Patentblatt 84/44**

(45) Bekanntmachung des Hinweises auf die Patenterteilung:
**28.01.87 Patentblatt 87/5**

(84) Benannte Vertragsstaaten:
**AT BE CH DE FR GB IT LI NL**

(56) Entgegenhaltungen:
**GB - A - 1 014 917**

**CHEMICAL ABSTRACTS, Band 86, Nr. 10, 7. März 1977, Seite 89, Nr. 56815n, Columbus, Ohio, USA W. UERDINGEN et al.: "New heterocyclic polyisocyanates"**

(73) Patentinhaber: **Diamalt Aktiengesellschaft, Friedrichstrasse 18, D-8000 München (DE)**

(72) Erfinder: **Mäke, Siegfried, Kufsteiner Strasse 109, D-8201 Raubling-Kirchdorf (DE)**
Erfinder: **Bauer, Adolf, Dr., Wendelsteinstrasse 6 C, D-8201 Raubling (DE)**
Erfinder: **Vogt, Hubert, Dr., König-Ludwig-Strasse 5 D, D-8201 Raubling (DE)**
Erfinder: **Wolf, Helmut, Dr., Haimgartenstrasse 6, D-8201 Neubeuern (DE)**

**Beschreibung**

Die Erfindung betrifft 4-Amino-3-imidazolin-2-on (= 4-Iminoimidazolidin-2-on und (2-Methoxy-2-imino-ethyl)-Harnstoff, ihre Herstellung und Verwendung.

Man erhält 4-Amino-3-imidazolin-2-on und (2-Methoxy-2-imino-ethyl)-Harnstoff, indem man Cyanomethylharnstoff in einem 1–4 Kohlenstoffatome enthaltenden Alkohol in Gegenwart katalytischer Mengen einer Base umsetzt. Geeignete Alkohole, in denen man das erfindungsgemässe Verfahren durchführt, sind beispielsweise Methanol, Ethanol oder Isopropanol. Die Reaktion wird vorzugsweise so durchgeführt, dass man 50 bis 1500 g und insbesondere 250 bis 1500 g Cyanomethylharnstoff im Liter Alkohol umsetzt. Geeignete Basen sind beispielsweise quartäre Ammoniumbasen wie Tetramethylammoniumhydroxid oder Cholin, Alkalimetallalkoholate wie Natrium- oder Kaliumalkoholat, welche sich von dem Alkohol ableiten, der als Lösungsmittel verwendet wird, oder Alkalimetall- oder Erdalkalimetallhydroxide wie z. B. Natriumhydroxid oder Kaliumhydroxid. Zur Durchführung der Reaktion verwendet man vorzugsweise pro mol Cyanomethylharnstoff 0,001 mol bis 0,2 mol Natriumhydroxid oder Kaliumhydroxid als Base. Vorzugsweise wird die Reaktion bei einer Temperatur von 0 °C bis 100 °C durchgeführt. Die Reaktionszeit beträgt bei Raumtemperatur etwa 0,2 bis 30 Stunden und verkürzt sich entsprechend, wenn man die Reaktion bei erhöhter Temperatur durchführt.

Führt man die Reaktion in Methanol bei Temperaturen von 10 bis 35 °C durch, so erhält man innerhalb von 0,2 bis 20 Stunden den (2-Methoxy-2-imino-ethyl)-Harnstoff. Führt man die Reaktion bei erhöhter Temperatur durch, oder verwendet man einen anderen Alkohol als Methanol, so entsteht das 4-Amino-3-imidazolin-2-on. Für den Fachmann war es überraschend, dass sich unter den erfindungsgemässen Verfahrensbedingungen das 4-Amino-3-imidazolin-2-on und/oder der (2-Methoxy-2-imino-ethyl-ethyl)-Harnstoff in hoher Ausbeute bilden würde, denn der Fachmann konnte nicht erwarten, dass der Cyanomethylharnstoff unter so milden Bedingungen reagiert. Ferner konnte der Fachmann nicht vorhersehen, dass der (2-Methoxy-2-imino-ethyl)-Harnstoff im Methanol und das 4-Amino-3-imidazolin-2-on in den übrigen Alkoholen so schwer löslich sein würde, dass sich das Reaktionsgleichgewicht zugunsten dieser Produkte verschieben würde. Aufgrund ihrer aktiven Methylengruppen eignen sich 4-Amino-3-imidazolin-2-on und (2-Methoxy-1-imino-ethyl)-Harnstoff vorzüglich als Komponente für Aldolkondensationsreaktionen und sind somit wertvolle Zwischenprodukte zur Synthese gewerblich verwertbarer Substanzen. So kann man 4-Amino-3-imidazolin-2-on und (2-Methoxy-2-imino-ethyl)-Harnstoff beispielsweise dazu verwenden, um aus ihnen Orotsäure herzustellen.

Die Synthese von Orotsäure aus 4-Amino-3-imidazolin-2-on oder (2-Methoxy-2-imino-ethyl)-Harnstoff zur Herstellung von Orotsäure gemäss Patentanspruch 6 kann nicht nur unter Verwendung der isolierten Substanzen selbst, sondern auch unter Verwendung von Reaktionsgemischen, die diese Substanzen enthalten, durchgeführt werden.

Vorzugsweise führt man die Orotsäuresynthese in wässriger oder wässrig-alkoholischer Phase durch, wobei man etwa 0,1 bis 5 mol 4-Amino-3-imidazolin-2-on oder (2-Methoxy-2-imino-ethyl)-Harnstoff pro Liter Lösungsmittel einsetzt. Pro mol 4-Amino-3-imidazolin-2-on oder (2-Methoxy-2-iminoethyl)-Harnstoff verwendet man 0,5 bis 2,0 mol Glyoxylsäure, vorzugsweise aber 0,8 bis 1,2 mol dieser Verbindung. Die Reaktion kann unter Verwendung der obengenannten basischen Katalysatoren durchgeführt werden. Vorzugsweise verwendet man Natriumhydroxid oder Kaliumhydroxid, die man so zu Reaktionsmischng zusetzt, dass am Ende der Reaktion eine 0,1 bis 5 n Basizität vorherrscht. Die Reaktionstemperatur beträgt vorzugsweise 20 bis 100 °C. Am Ende der Reaktion wird entweder neutralisiert, so dass man das Natriumsalz der Orotsäure erhält, oder man säuert stark an und erhält das Orotsäuremonohydrat.

Gegenüber dem aus der Deutschen Patentschrift 2502951 vorbekannten Verfahren zeichnet sich die erfindungsgemässe Herstellungsweise für Orotsäure unter anderem durch eine hervorragende Reinheit des Verfahrensprodukts aus. Dies ist von besonderer Bedeutung, da die Orotsäure durch Umkristallisation nur schwer gereinigt werden kann.

Die nachfolgenden Ausführungsbeispiele dienen zur Erläuterung des erfindungsgemässen Verfahrens.

Beispiel 1

Eine Suspension von 198 g Cyanomethylharnstoff in 300 ml Ethanol wird unter Rückfluss erhitzt, mit 6 ml 8 n Natronlauge (bzw. mit 7 ml 7 n Kalilauge) versetzt und nach dem Abklingen der exothermen Reaktion weitere 5 Minuten erhitzt. Dann lässt man die Reaktionsmischung erkalten, filtriert das abgeschiedene Produkt ab, wäscht es mit wenig eisgekühltem Ethanol, trocknet es und erhält 194 g 4-Amino-3-imidazolin-2-on (= 98% der Theorie).

Praktisch die gleichen Ausbeuten werden erhalten, wenn man anstelle von Ethanol Methanol oder Isopropanol verwendet.

Beispiel 2

100 g Cyanomethylharnstoff werden mit einem Gemisch von 300 ml Methanol und 5 ml 8 n Natronlauge versetzt und 3 Stunden lang bei 25 bis 30 °C gerührt. Man lässt die Mischung auf 20 °C abkühlen, filtriert das erhaltene Produkt ab, wäscht es mit wenig eiskaltem Methanol, trocknet es im Vakuum und erhält 92,6 g (2-Methoxy-2-imino-ethyl)-Harnstoff (= 70% der Theorie).

Beispiel 3

26 g (2-Methoxy-2-imino-ethyl)-Harnstoff werden mit einem Gemisch aus 50 ml Ethanol und

1 ml 8 n Natronlauge versetzt und 30 Minuten lang unter Rückfluss erhitzt. Man arbeitet die Reaktionsmischung auf, wie in Beispiel 1 beschrieben, und erhält 18,6 g 4-Amino-3-imidazolin-2-on (= 94,7 % der Theorie).

Charakterisierung der erfindungsgemässen Substanzen im Vergleich mit Cyanomethylharnstoff

| | Fp °C | N ber. (%) | N gef. (%) | Löslichkeit in 100 ml $H_2O$ bei Raumtemperatur (g) | λ max. |
|---|---|---|---|---|---|
| Cyanomethylharnstoff $C_3H_5N_3O$; 99,09 | 140–142 | 42,40 | 42,32 | 4,3 | 198 nm |
| 4-Amino-3-imidazolin-2-on $C_3H_5N_3O$; 99,09 | — (Z > 350 °C) | 42,40 | 42,35 | 0,4 | 230 nm |
| (2-Methoxy-2-iminoethyl)-Harnstoff $C_4H_9N_3O_2$; 131,14 | 108–110 (Z) | 32,04 | 31,97 | 9,8 | 198 nm |

Zur weiteren Charakterisierung dienen die IR-Spektren.

**Beispiel 4**

Zu 5 g Amino-2-imidazolin-2-on und 4,6 g Glyoxylsäure in 200 ml Wasser tropft man bei 60 °C innerhalb von 1 Stunde 62,5 ml 8 n Natronlauge zu und rührt weitere 2 Stunden bei dieser Temperatur nach. Anschliessend stellt man mit konzentrierter Salzsäure den pH-Wert auf 6, wobei Natriumorotat ausfällt. Nach Absaugen, Waschen und Trocknen erhält man 6,9 g dieses Salzes = 78 % der Theorie.

Gibt man alternativ nach beendeter Reaktion einen Salzsäureüberschuss bis zu einem pH 1 zu, so fällt des Orotsäuremonohydrat aus. Nach Isolieren und Trocknen bei mässiger Temperatur erhält man 6,8 g des Monohydrats, entsprechend 78 % der Theorie.
Die Identität der Verbindungen wird anhand der IR-Spektren nachgewiesen.

**Beispiel 5**

Eine Suspension von 57 g Cyanomethylharnstoff in 45 ml Methanol wird unter Rühren zum Sieden erhitzt, mit 3 ml 8 n Natronlauge versetzt und 30 Minuten lang unter Rückfluss erhitzt. Dann lässt man die Reaktionsmischung auf 30 °C erkalten, setzt ihr 550 ml Wasser und 90 g 50%ige Glyoxylsäure zu und neutralisiert bei einer Temperatur von max. 30 °C mit 8 n Natronlauge, bis ein pH von 6,5 erreicht ist. Dann erhitzt man die Mischung auf 55 bis 60 °C, tropft innerhalb einer Stunde bei dieser Temperatur 225 ml 8 n Natronlauge zu und rührt das Gemisch weitere 2 Stunden lang. Anschliessend destilliert man bis 95 °C ca. 150 ml wässriges Methanol ab, erhitzt noch eine Stunde unter Rückfluss und säuert das Gemisch vorsichtig mit konzentrierter Salpetersäure an. Man lässt die Mischung auf Raumtemperatur abkühlen, filtriert das abgeschiedene Kristallisat ab, wäscht es mit Wasser, trocknet es im Vakuum und erhält so 72 g Orotsäure (= 71,8 % der Theorie).

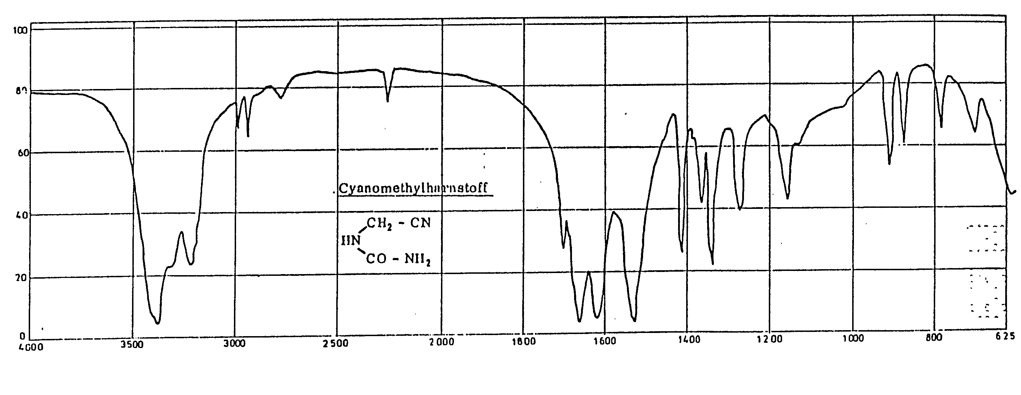

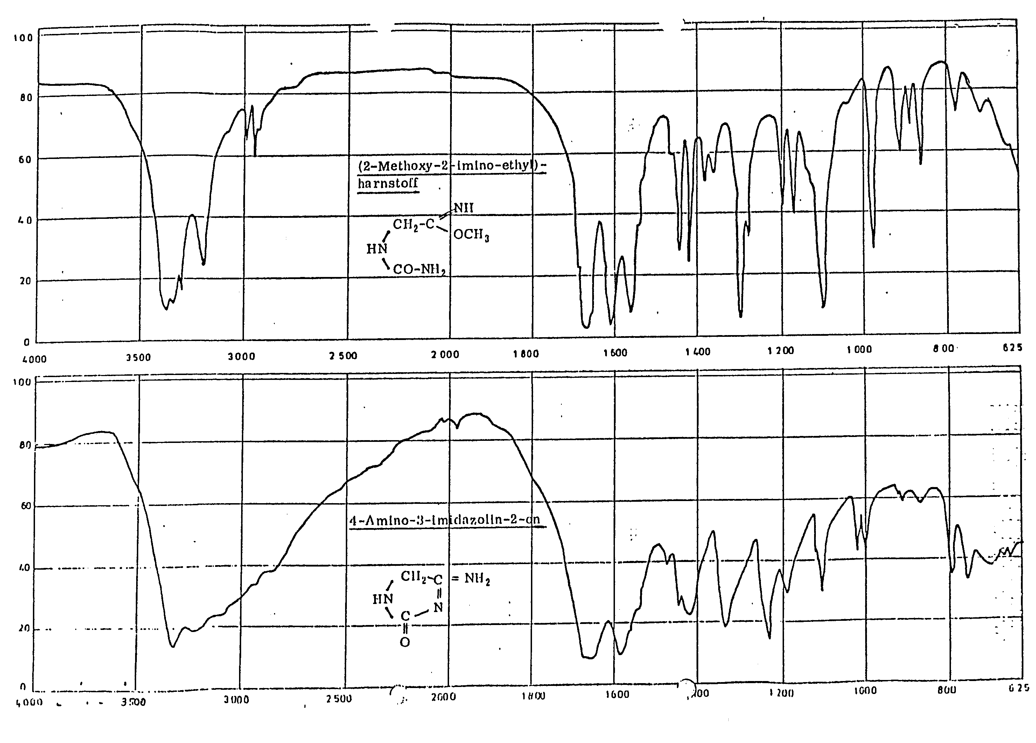

**Patentansprüche**

1. 4-Amino-3-imidazolin-2-on.

2. (2-Methoxy-2-imino-ethyl)-Harnstoff.

3. Verfahren zur Herstellung von 4-Amino-3-imidazolin-2-on, dadurch gekennzeichnet, dass man Cyanomethylharnstoff in einem 2 bis 4 Kohlenstoffatome enthaltenden Alkohol bei einer Temperatur von 0 °C bis bis 100 °C oder in Methanol bei einer Temperatur oberhalb 35 °C in Gegenwart einer katalytischen Menge einer Base umsetzt.

4. Verfahren zur Herstellung von 4-Amino-3-imidazolin-2-on gemäss Patentanspruch 3, dadurch gekennzeichnet, dass man pro Mol Cyanomethylharnstoff 0,001 mol bis 0,2 mol Natriumhydroxid oder Kaliumhydroxid verwendet.

5. Verfahren zur Herstellung von (2-Methoxy-2-imino-ethyl)-Harnstoff, dadurch gekennzeichnet, dass man Cyanomethylharnstoff in Methanol bei einer Temperatur von 10 bis 35 °C in Gegenwart einer katalytischen Menge einer Base umsetzt.

6. Verfahren zur Herstellung von (2-Methoxy-2-imino-ethyl)-Harnstoff gemäss Patentanspruch 5, dadurch gekennzeichnet, dass man pro Mol Cyanomethylharnstoff 0,001 mol bis 0,2 mol Natriumhydroxid oder Kaliumhydroxid verwendet.

7. Verfahren zur Herstellung von Orotsäure, dadurch gekennzeichnet, dass man 4-Amino-3-imidazolin-2-on oder (2-Methoxy-2-imino-ethyl)-Harnstoff in Gegenwart basischer Katalysatoren mit Glyoxylsäure umsetzt.

8. Verfahren zur Herstellung von 4-Amino-3-imidazolin-2-on, dadurch gekennzeichnet, dass man (2-Methoxy-2-imino-ethyl)-Harnstoff in einem 2 bis 4 Kohlenstoffatome enthaltenden Alkohol in Gegenwart einer katalytischen Menge einer Base umsetzt.

**Claims**

1. 4-Amino-3-imidazolidin-2-one.

2. (2-Methoxy-2-iminoethyl)urea.

3. Process for the preparation of 4-amino-3-imidazolidin-2-one, characterised in that cyanomethylurea is reacted in an alcohol of 2 to 4 carbon atoms at a temperature of from 0 °C to 100 °C or in methanol at a temperature of above 35 °C in the presence of a catalytic amount of base.

4. Process for the preparation of 4-amino-3-imidazolidin-one according to claim 3, characterised in that 0.001 to 0.2 moles of sodium hydroxide or potassium hydroxide are employed per mole of cyanomethylurea.

5. Process for the preparation of (2-methoxy-2-iminoethyl)urea, characterised in that cyanomethylurea is reacted in methanol at a temperature of from 10 to 35 °C in the presence of a catalytic amount of a base.

6. Process for the preparation of (2-methoxy-2-iminoethyl)urea according to claim 5, characterised in that 0.001 to 0,2 moles of sodium hydroxide or potassium hydroxide are employed per mole of cyanomethylurea.

7. Process for the preparation of orotic acid, characterised in that 4-amino-3-imidazolidin-2-one or (2-methoxy-2-iminoethyl)urea is reacted in the presence of a basic catalyst with glyoxalic acid.

8. Process for the prepatation of 4-amino-3-imidazolidin-2-one, characterised in that (2-methoxy-2-iminoethyl)urea is reacted in an alcohol of 2 to 4 carbon atoms in the presence of a catalytic amount of a base.

**Revendications**

1. Amino-4 $\Delta^3$-imidazolinone-2.

2. (Méthoxy-2 imino-2 éthyl)-urée.

3. Procédé de préparation de l'amino-4 $\Delta^3$-imidazolinone-2 caractérisé en ce qu'on fait réagir la cyanométhyl-urée dans un alcool contenant de 2 à 4 atomes de carbone à une température de 0 à 100 °C ou dans le méthanol à une température supérieure à 35 °C, en présence d'une quantité catalytique d'une base.

4. Procédé de préparation de l'amino-4 $\Delta^3$-imidazolinone-2 selon la revendication 3, procédé caractérisé en ce qu'on utilise, par mole de cyanométhyl-urée, de 0,001 à 0,2 mol d'hydroxyde de sodium ou d'hydroxyde de potassium.

5. Procédé de préparation de la (méthoxy-2 imino-2 éthyl)-urée caractérisé en ce qu'on fait réagir la cyanométhyl-urée dans du méthanol, à une température de 10 à 35 °C, en présence d'une quantité catalytique d'une base.

6. Procédé de préparation de la (méthoxy-2 imino-2 éthyl)-urée selon la revendication 5, procédé caractérisé en ce qu'on utilise, par mole de cyanométhyl-urée, de 0,001 mol à 0,2 mol d'hydroxyde de sodium ou d'hydroxyde de potassium.

7. Procédé de préparation de l'acide orotique caractérisé en ce qu'on fait réagir l'amino-4 $\Delta^3$-imidazolinone-2 ou la (méthoxy-2 imino-2 éthyl)-urée, en présence de catalyseurs basiques, avec l'acide glyoxylique.

8. Procédé de préparation de l'amino-4 $\Delta^3$-imidazolinone-2 caractérisé en ce qu'on fait réagir la (méthoxy-2 imino-2 éthyl)-urée dans un alcool contenant de 2 à 4 atomes de carbone en présence d'une quantité catalytique d'une base.

Cyanomethylharnstoff

$$CH_2 - CN$$
$$HN$$
$$CO - NH_2$$

0 123035

(2-Methoxy-2-imino-ethyl)-harnstoff

$$CH_2-C \begin{cases} NH \\ OCH_3 \end{cases}$$

HN
CO-NH₂

4-Amino-3-imidazolin-2-on

$CH_2-C = NH_2$

HN
C    N
O